# EUROPÄISCHE PATENTSCHRIFT

(11) **EP 0 911 024 B1**
(45) Veröffentlichungstag und Bekanntmachung des Hinweises auf die Patenterteilung: **04.02.2004**
(21) Anmeldenummer: 98118228.0
(22) Anmeldetag: 25.09.1998
(51) Int. Cl.: A61K 9/28, A61K 47/34

(54) **Verwendung von wasserlöslichen oder wasserdispergierbaren Polyurethanen als Überzugsmittel oder Bindemittel für pharmazeutische Darreichungsformen**
Use of water-soluble or water-dispersible polyurethans as coating agents or binders for pharmaceutical administration forms
Utilisation de polyuréthanes solubles ou dispersibles dans l'eau comme agents de revêtement ou liants pour formes d'administration pharmaceutiques

(30) Priorität: 09.10.1997 DE 19744473
(43) Veröffentlichungstag der Anmeldung: 28.04.1999
(73) Patentinhaber: BASF AKTIENGESELLSCHAFT, 67056 Ludwigshafen (DE)
(72) Erfinder: Nguyen Kim, Son, Dr., 69502 Hemsbach (DE); Sanner, Axel, Dr., 67227 Frankenthal (DE); Kolter, Karl, Dr., 67117 Limburgerhof (DE)

(56) Entgegenhaltungen:
- DE-A- 4 225 045
- DE-A- 4 241 118
- DE-A- 19 541 326

## Beschreibung

Die vorliegende Erfindung betrifft die Verwendung von wasserlöslichen oder wasserdispergierbaren Polyurethanen als Überzugsmittel oder Bindemittel für pharmazeutische Darreichungsformen.

Feste pharmazeutische Darreichungsformen wie Tabletten, Kapseln, Pellets, Granulate, Kristalle etc. werden aus sehr unterschiedlichen Gründen gecoatet, d. h. mit einem Filmüberzug versehen. So kann beispielsweise ein schlechter Geruch oder Geschmack maskiert sowie die Schluckbarkeit verbessert werden. Die Stabilität des Wirkstoffes kann durch das Coating erhöht werden, indem weniger Wasserdampf und Sauerstoff an das Tabletteninnere gelangt. Die Darreichungsformen sehen besser aus und können durch die Einarbeitung von Farbstoffen besser unterschieden werden. Darüberhinaus läßt sich insbesondere die Freisetzungsgeschwindigkeit des Wirkstoffes durch den Filmüberzug einstellen.

Generell unterscheidet man Instant-Release-Formen und Retard- bzw. Slow-Release-Formen.

Bei Instant-Release-Formen sollen der Zerfall der Tablette und die Freisetzung des Wirkstoffs aus der Darreichungsform nach Möglichkeit nicht durch das Coating beeinflußt werden, deshalb muß sich der Filmüberzug schnell in Magensaft auflösen. Daneben muß er über gute Filmeigenschaften verfügen. Die Zugfestigkeit und die Reißdehnung sollten hoch sein, damit der Filmüberzug mechanischen Einwirkungen standhält, wie sie bei der pharmazeutischen Verarbeitung - insbesondere der Konfektionierung - und auch während des Versandes bzw. der Lagerung auftreten.

Ein häufig eingesetztes Produkt für das Coaten von Instant-Release-Tabletten ist Hydroxypropylmethylcellulose (HPMC). Hydroxypropylmethylcellulose weist in wäßriger Lösung bei zunehmender Konzentration einen steilen Viskositätsanstieg auf.

Da die Filmbildnerlösung beim Coaten von Tabletten fein zerstäubt werden muß und die gebildeten Tröpfchen die Oberfläche der Tabletten gut benetzen und auch gut spreiten müssen, darf die Viskosität eine gewisse Grenze (zwischen 150 und 250 mPas), die abhängig ist von der Art der Sprühdüse und des Gerätes, nicht überschreiten. Deshalb können im Falle von HPMC nur verhältnismäßig niedrige Filmbildnerkonzentrationen eingesetzt werden.

Als Empfehlung für die Konzentration von Pharmacoat® 606 (Fa. Shin-etsu) werden in der Literatur 5-7 Gew.-% angegeben (Pharmaceutical Coating Technology, edited by Graham Cole, Taylor and Francis Ltd. 1995 und Technische Merkblätter der Hersteller). Diese geringe Sprühkonzentrationen bedingen relativ lange Verarbeitungszeiten und damit hohe Kosten.

Darüber hinaus zeigt Hydroxypropylmethylcellulose weitere Nachteile u.a. im Benetzungsverhalten, in der Adhesivität auf der Tablettenoberfläche, im Pigmentbindevermögen, in den mechanischen Eigenschaften der Filme, in der Hygroskopizität sowie in der Permeabilität gegenüber Wasserdampf und Sauerstoff und in der Zerfallszeitdifferenz zwischen Filmtabletten und Kern.

Der vorliegenden Erfindung lag die Aufgabe zugrunde, wasserlösliche oder wasserdispergierbare Polyurethane als Überzugsmittel oder Bindemittel für pharmazeutische Darreichungsformen bereitzustellen, die die oben genannten Nachteile nicht aufweisen.

Die Aufgabe wurde erfindungsgemäß gelöst durch Verwendung von wasserlöslichen oder wasserdispergierbaren Polyurethanen aus
a) 0,1 bis 30 Gew.-% mindestens eines Polyols,
b) 20 bis 45 Gew.-% mindestens eines Polyetherpolyols,
c) 10 bis 45 Gew.-% mindestens eines, eines sulfonsäure haltigen Diamins
d) 30 bis 50 Gew.-% mindestens eines Polyisocyanats sowie gegebenenfalls
e) weiteren Zusatzstoffen.

Polyurethane sowie Polyester als auch Poly(ester-urethane) als Hilfsmittel in pharmazeutischen Zubereitungen sind bereits bekannt.

So beschreibt DE-A-42 25 045 die Verwendung von wasserlöslichen oder in Wasser dispergierbaren Polyurethanen aus mindestens einer Verbindung, die zwei oder mehrere aktive Wasserstoffatome pro Molekül enthält, aus einem Diisocyanat und einem Säure- oder Salzgruppen enthaltendem Diol. Die Carboxylatgruppen enthaltenden Diole haben den Nachteil, daß sie das Polymer magensaftunlöslich machen.

Die in DE-A-42 41 118 beschriebenen Polyurethane aus Polyesterdiolen/Diolen/tert. aminhaltigen Diolen o. Diaminen/Diisocyanaten sind im sauren pH-Bereich hydrolyselabil und neigen zur Ausbildung klebriger Filme.

DE-A-43 33 238 beansprucht pyrrolidongruppenhaltige Polyester und Polyamide als Bestandteil von Filmüberzügen in pharmazeutischen Zubereitungen. Die hier beschriebenen Polyester sind ebenfalls unter magensauren Bedingungen zu hydrolyselabil. Die genannten Polyamide haben den Nachteil, daß sie zu viskos und zu klebrig sind, und daher nicht die für Filmbildner gewünschten Eigenschaften aufweisen.

In DE-A-31 51 923 werden vernetzte Polyetherurethane beschrieben, die als Tablettierhilfsmittel eingesetzt werden können. Um jedoch die gewünschte Wasserlöslichkeit der vernetzten Polyetherurethane zu erzielen, muß der Polyetheranteil so hoch gewählt werden, daß das Polymer zu klebrig wird.

In den US-Patenten 4,743,673; 3,388,159 und 4,789,720 werden Polyurethane beschrieben, die entweder wasserunlöslich sind, oder unerwünscht klebrige Filme bilden.

Die erfindungsgemäßen Polyurethane beziehungsweise deren Aufbaukomponenten a) bis d) lassen sich im einzelnen wie folgt beschreiben.

Bei den als Komponente a) verwendeten Polyolen handelt es sich um niedermolekulare Polyole, insbesondere um 2 bis 10 C-Atome enthaltende Diole mit einem MG ≤ 500 g/mol. Als bevorzugte Vertreter dieser Klasse kommen Ethylenglykol, Propylenglykol-(1,2) und Propylenglykol-(1,3), Butandiol-(1,4), Butandiol-(1,3), Butendiol-(1,4), Butindiol-(1,4), Pentandiol-(1,5), Hexandiol-(1,6), Octandiol-(1,8), Neopentylglykol, Cyclohexandimethanol (1,4-Bishydroxymethylcyclohexan), 2-Methyl-1,3-propandiol, ferner Diethylenglykol, Triethylenglykol, Tetraethylenglykol, Dipropylenglykol und Dibutylenglykol in Frage. Auch Triole wie Glycerin kommen in Betracht.

Besonders bevorzugte Diole sind Hexandiol-(1,6) sowie 1,4-Bishydroxymethylcyclohexan. Die Mengen an Komponente a) im erfindungsgemäßen Polyurethan liegen im Bereich von 0,1 bis 30 Gew.-%, bevorzugt im Bereich von 0,3 bis 20 Gew.-%, besonders bevorzugt im Bereich von 0,5 bis 15 Gew.-%.

Bei den Komponenten b) handelt es sich um Polyetherpolyole, insbesondere Polyetherdiole mit einem Molekulargewicht von 300 bis 6000 g/mol, vorzugsweise 500 bis 4000 g/mol, besonders bevorzugt 500 bis 2000 g/mol.

Die einsetzbaren Polyetherdiole sind insbesondere durch Polymerisation von Ethylenoxid, Propylenoxid, Butylenoxid, Tetrahydrofuran, Styroloxid oder Epichlorhydrin mit sich selbst, z.B. in Gegenwart von BF₃ oder durch Anlagerung dieser Verbindungen gegebenenfalls im Gemisch oder nacheinander, an Startkomponenten mit reaktionsfähigen wasserstoffatomen, wie Alkohole oder Amine, z.B. Wasser, Ethylenglykol, Propylenglykol-(1,3) oder Propylenglykol-(1,2), 4,4'-Dihydroxydiphenylpropan oder Anilin erhältlich.

Bevorzugte Polyetherdiole sind Polytetrahydrofuran mit einem Molekulargewicht von 500 bis 2000 g/mol sowie Polyethylenglykol und Polypropylenglykol mit einem Molekulargewicht von 500 bis 2000 g/mol.

Es ist auch möglich, bis zu 30 mol-% der Komponente b) durch ein Polymilchsäureesterdiol zu ersetzen. Diese Polymilchsäureesterdiole haben den Vorteil, daß sie bei der Hydrolyse, beispielsweise im Magensaft, toxikologisch unbedenkliche Monomere bilden.

Die Mengen an Komponente b) im erfindungsgemäßen Polyurethan liegen im Bereich von 20 bis 45 Gew.-%, bevorzugt im Bereich von 22 bis 40 Gew.-%, besonders bevorzugt im Bereich von 23 bis 35 Gew.-%.

Als weitere Komponente c) sind Verbindungen gemeint, die mindestens zwei gegenüber Isocyanatgruppen reaktionsfähige Amino-Gruppen und außerdem ionische Gruppen oder durch eine einfache Neutralisations- oder Quarternisierungsreaktion in ionische Gruppen überführbare, potentiell ionische Gruppen aufweisen. Durch Einführung der Monomeren c) werden die Polyurethane selbstdispergierbar, d.h. beim Dispergieren in Wasser werden in diesem Fall keine Dispergierhilfsmittel wie Schutzkolloide oder Emulgatoren benötigt.

Die Einführung der kationischen oder anionischen Gruppen kann durch Mitverwendung von (potentiell) kationische oder (potentiell) anionische Gruppen aufweisenden Diaminen erfolgen.

Vorzugsweise werden jedoch die ionischen Gruppen durch Mitverwendung von vergleichsweise niedermolekularen Verbindungen, insbesondere Diamine mit einem Molekulargewicht unter 500 g/mol, mit (potentiell) ionischen Gruppen eingeführt.

Bevorzugt sind Sulfonsäure-haltige Diamine und deren Salze, wie z.B. N-(2-Aminoethyl)-2-aminoethansulfonsäure und dessen Na, K oder Ammoniumsalze.

Die Überführung der gegebenenfalls zunächst in das Polyadditionsprodukt eingebauten potentiell ionischen Gruppen in zumindest teilweise ionische Gruppen geschieht in an sich üblicher Weise durch Neutralisation der potentiell anionischen oder kationischen Gruppen oder durch Quarternierung von tertiären Amin-Stickstoffatomen.

Zur Neutralisation von potentiell anionischen Gruppen, z.B. Carboxylgruppen oder bevorzugt Sulfonatgruppen, werden anorganische und/oder organische Basen eingesetzt, wie Alkalihydroxide, -carbonate oder -hydrogencarbonate, Ammoniak oder primäre, sekundäre und besonders bevorzugt tertiäre Amine wie Triethylamin oder Dimethylaminopropanol.

Zur Überführung der potentiell kationischen Gruppen, z.B. der tertiären Amingruppen in die entsprechenden Kationen, z.B. Ammoniumgruppen, sind als Neutralisationsmittel anorganische oder organische Säuren, z.B. Salz-, Phosphor-, Ameisen-, Essig-, Fumar-, Malein-, Milch-, Wein- oder Oxalsäure oder als Quarternierungsmittel z.B. Methylchlorid, Methylbromid, Methyljodid, Dimethylsulfat, Benzylchlorid, Chloressigsäureester oder Bromacetamid geeignet. Weitere Neutralisations- oder Quarternierungsmittel sind z.B. in der US 3,479,310 (Spalte 6) beschrieben.

Diese Neutralisation oder Quarternierung der potentiellen Ionengruppen kann vor, während, jedoch vorzugsweise nach der Isocyanat-Polyadditionsreaktion erfolgen.

Die Mengen an Komponente c) im erfindungsgemäßen Polyurethan liegen im Bereich von 10 bis 45 Gew.-%, bevorzugt im Bereich von 15 bis 43 Gew.-%, besonders bevorzugt im Bereich von 20 bis 43 Gew.-%.

Als Polyisocyanate (Komponente d) zu nennen sind insbesondere Diisocyanate X(NCO)₂, wobei X für einen aliphatischen Kohlenwasserstoffrest mit 4 bis 12 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht. Beispiele derartiger Diisocyanate sind Tetramethylendiisocyanat, Hexamethylendiisocyanat, Dodecamethylendiisocyanat, 1,4-Diisocyanatocyclohexan, 1-Isocyanato-3,5,5-trimethyl-3-isocyanatomethylcyclohexan (Isophorondiisocyanat), 2,2-Di-(4-isocyanatocyclohexyl)propan, Trimethylhexandiisocyanat, 1,4-Diisocyanatobenzol, 2,4-Diisocyanatotoluol, 2,6-Diisocyanatotoluol, 4,4'-Diisocyanato-diphenylmethan, p-Xylylendiisocyanat, m- oder p-Tetramethylxylylendiisocyanat sowie aus diesen Verbindungen bestehende Gemische. Auch monofunktionelle oder höher als difunktionelle Isocyanate können in kleinen Anteilen mitverwendet werden.

Bevorzugt verwendete Diisocyanate sind Isophorondiisocyanat und Hexamethylendiisocyanat.

Es ist auch möglich, bis zu 10 mol-% der o.g. Diisocyanate durch Polyisocyanate, wie z.B. Basocyanat® HI 100 (Fa. BASF) zu ersetzen.

Die Mengen an Komponente d) im erfindungsgemäßen Polyurethan liegen im Bereich von 30 bis 50 Gew.-%, bevorzugt im Bereich von 32 bis 48 Gew.-%, besonders bevorzugt im Bereich von 32 bis 45 Gew.-%.

Als weitere Zusatzstoffe (Komponente e) können gegebenenfalls Emulgatoren oder Schutzkolloide mitverwendet werden.

Als Emulgatoren bzw. Schutzkolloide zu nennen sind beispielsweise Alkalisalze von längerkettigen Fettsäuren, Alkylsulfate und -sulfonate, alkylierte Arylsulfonate oder alkylierte Biphenylethersulfonate. Des weiteren kommen als Emulgatoren Umsetzungsprodukte von Alkylenoxiden, insbesondere Ethylen- oder Propylenoxid, mit Fettalkoholen, Fettsäuren, Phenol oder Alkylphenolen in Betracht.

Als Schutzkolloide können z.B. Polyvinylalkohol, Polyvinylpyrrolidon, Methylcellulose, Carboxymethylcellulose, Hydroxypropylcellulose, Hydroxypropylmethylcellulose, Stärke, Gelatine, Kasein u.ä. verwendet werden.

Die K-Werte der Polymerisate sollen im Bereich von 20 bis 150, vorzugsweise 25 bis 120, besonders bevorzugt im Bereich von 30 bis 80 liegen. Der jeweils gewünschte K-Wert läßt sich in an sich bekannter Weise durch die Zusammensetzung der Einsatzstoffe einstellen. Die K-Werte werden nach Fikentscher, Cellulosechemie, Bd. 13, S. 58-64 und 71-74 (1932) bei 25°C in 0,1 Gew.-%iger Lösung in N-Methylpyrrolidon gemessen.

Die Säurezahl (bzw. die korrespondierende Salzzahl) der erfindungsgemäßen Polyurethane wird von der Zusammensetzung und der Konzentration der Verbindungen der Komponente c) bestimmt. Die Werte für die Säurezahl liegen zwischen 30 und 120, insbesondere zwischen 40 und 90.

Die Herstellung der erfindungsgemäßen wasserlöslichen oder wasserdispergierbaren Polyurethane erfolgt zweckmäßig so, daß man die Monomeren a, b und d in Gegenwart eines inerten, mit Wasser mischbaren Lösungsmittel zu Polyurethanprepolymeren umsetzt.

Als organische Lösungsmittel eignen sich im Prinzip alle aprotischen organischen Lösungsmittel, vor allem solche mit einem Siedepunkt von 40 bis 90°C, bei Normaldruck. Zu erwähnen sind hier beispielsweise Tetrahydrofuran, Essigethylester und vor allem Methylethylketon und Aceton.

Im allgemeinen werden in den obengenannten Lösungsmitteln die Aufbaukomponenten der Polyurethane bei Temperaturen von 20 bis 160°C, insbesondere von 40 bis 90°C umgesetzt.

Zur Beschleunigung der Reaktion der Diisocyanate können die üblichen Katalysatoren, z.B. Dibutylzinndilaurat, Zinn-II-octoat oder Diazabicyclo-(2,2,2)-octan, mitverwendet werden.

Der Feststoffgehalt der erhaltenen organischen Lösung vor dem Dispergieren in Wasser beträgt üblicherweise 20 bis 90 Gew.-%, insbesondere 50 bis 80 Gew.-%.

Die organische Lösung von Polyurethanpräpolymeren, d.h. Polyurethanen, welche noch freie Isocyanatgruppen enthalten, wird mit den aminofunktionellen Komponenten c), gegebenenfalls in Gegenwart von Wasser zum Endprodukt umgesetzt.

Die Überführung potentieller Salzgruppen, z.B. Sulfonsäuregruppen oder tertiärer Aminogruppen, welche über die Monomeren c) in das Polyurethan eingeführt wurden, in die entsprechenden Ionen erfolgt durch Neutralisation mit Basen oder Säuren oder durch Quarternisierung der tertiären Aminogruppen vor oder während dem Dispergieren der Polyurethane in Wasser.

Der Feststoffgehalt der erhaltenen wäßrigen Polyurethan-Dispersionen bzw. Lösungen beträgt in der Regel 10 bis 70 Gew.-%, insbesondere 15 bis 50 Gew.-%.

Die Polyurethan-Dispersionen oder Lösungen können durch verschiedene Trocknungsverfahren wie z.B. Sprühtrocknung, Fluidized Spray Drying, Walzentrocknung oder Gefriertrocknung in Pulverform überführt werden, aus der sich durch Redispergieren in Wasser erneut eine wäßrige Dispersion bzw. Lösung herstellen läßt.

Die erfindungsgemäßen wasserlöslichen oder wasserdispergierbaren Polyurethane eignen sich hervorragend als magensaftlösliche oder als im Magensaft dispergierbare Filmbildner und/oder Bindemittel für pharmazeutische Darreichungsformen.

Gegenstand der Erfindung sind daher auch feste pharmazeutische Darreichungsformen mit einem Überzugsmittel oder Bindemittel aus
a) 0,1 bis 30 Gew.-% mindestens eines Polyols,
b) 20 bis 45 Gew.-% mindestens eines Polyetherpolyols,
c) 10 bis 45 Gew.-% mindestens eines sulfonsäure haltigen Diamins
d) 30 bis 50 Gew.-% mindestens eines Polyisocyanats sowie gegebenenfalls
e) weiteren Zusatzstoffen,
wobei die Komponenten a) bis e) die bereits oben genannte Bedeutung haben können.

Bei den überzogenen Darreichungsformen handelt es sich bevorzugt u.a. um Filmtabletten, Filmmikrotabletten, Dragees, überzogene Pastillen, Kapseln, Kristalle, Granulate oder Pellets.

Bei den bindemittelhaltigen Darreichungsformen handelt es sich bevorzugt u.a. um Tabletten, Mikrotabletten, Kerne, Granulate oder Pellets.

Wie Tabelle 1 zeigt, weisen die wäßrigen Lösungen der erfindungsgemäßen Polyurethane eine deutlich niedrigere Viskosität auf als entsprechende Lösungen von Hydroxypropylmethylcellulose.

**Tabelle 1:**

| | Viskosität (30 Gew.-%ige wäßr. Lösung) [mPas] | Viskosität (20 Gew.-%ige wäßr. Lösung) [mPas] |
|---|---|---|
| | | |
| Polyurethan A ¹⁾ | 8 | - |
| Polyurethan B ²⁾ | 40 | - |
| Polyurethan C ³⁾ | 21 | - |
| Pharmacoat® 606 | - | > 2000 |
| | | |

| | | |
|---|---|---|
| Zusammensetzung (Gew.-%): ¹⁾ 31,35 % Poly THF (MG 650), 0,71 % Hexandiol 1,6, 40,21 % Iso-phorondiisocyanat, 27,73 % N-(2-Aminoethyl)-2-aminoethansulfonsäure Na-Salz | | |
| ²⁾ 23,15 % Poly THF (MG 650), 0,95 % Hexandiol 1,6, 33,60 % Iso-phorondiisocyanat, 42,30 % N-(2-Aminoethyl)-2-aminoethansulfonsäure Na-Salz | | |
| ³⁾ 29,46 % Poly THF (MG 650), 1,48 % 1,4-Cyclohexyldimethylol, 41,56 % Isophorondiisocyanat, 27,5 % N-(2-Aminoethyl)-2-aminoethansulfonsäure Na-Salz | | |

Somit können beim Coaten von Tabletten mit den Polyurethandispersionen ebenso wie bei Bindemittelanwendungen konzentriertere Polymerzubereitungen eingesetzt werden, wodurch sich die Verfahren wesentlich kostengünstiger und zeitsparender gestalten lassen.

Die Redispergierung der pulver- oder granulatförmigen Polyurethane zu wäßrigen Dispersionen bzw. Lösungen erfolgt wesentlich schneller als bei anderen Filmbildnern oder Bindemitteln, da die Polyurethane gut von Wasser benetzt werden, wenig klumpen und eine sehr hohe Auflösungsgeschwindigkeit aufweisen. Wäßrige Dispersionen der erfindungsgemäßen Polyurethane sind außerordentlich scherstabil und übertreffen die handelsüblichen pharmazeutischen Dispersionen wie z.B. Acrylat-Methacrylat-Dispersionen bei weitem.

Magensaftlösliche Tabletten, die mit den erfindungsgemäßen Polyurethanen gecoatet wurden, zeigen eine gegenüber dem Kern nur geringfügig verlängerte Zerfallszeit, d. h. der Filmüberzug löst sich sehr schnell in künstlichem Magensaft auf.

Im Fall von Hydroxypropylmethylcellulose, Typ Pharmacoat 606 als Coatingmaterial ist der Zerfall deutlich verlängert (Siehe Beispiele 2, 3 und Vergleichsbeispiel). Ferner wird durch die erfindungsgemäße Verwendung der Polyurethane die mechanische Festigkeit der Tabletten im Vergleich zu Hydroxypropylmethylcellulose sehr viel stärker erhöht.

Tabletten quellen in Abhängigkeit von den verwendeten Hilfs- und Wirkstoffen, der Lagerzeit und den Lagerbedingungen, wie Temperatur und Feuchtigkeit, unterschiedlich stark. Ein starrer Filmüberzug erleidet bei Quellung des Kernes Risse. Deshalb ist die Elastizität von Filmbildnern eine wichtige Größe. Polyurethane besitzen eine ausgesprochen hohe Flexibilität und Elastizität. So kann die Reißdehnung bis zu 300 % betragen. Eine Rißbildung ist daher auch bei starker Kernquellung nicht zu erwarten.

Die Polyurethane können in reiner Form oder aber zusammen mit den üblichen Hilfsstoffen auf den wirkstoffhaltigen Kern appliziert werden. Übliche Hilfsstoffe sind z. B. Farbpigmente zur Einfärbung, Weißpigmente, wie Titandioxid, zur Erhöhung der Deckkraft, Talkum und Siliciumdioxid als Antiklebemittel, Polyethylenglykole, Glycerin, Propylenglykol, Triacetin, Triethylcitrat als Weichmacher und verschiedene oberflächenaktive Stoffe, wie Natriumlaurylsulfat, Polysorbat 80, Pluronics und Cremophore, zur Verbesserung des Benetzungsverhaltens. Die exemplarisch genannten Stoffe stellen keine Begrenzung dar. Es können alle bekanntermaßen für magensaftlösliche Filmüberzüge geeigneten Zusatzstoffe verwendet werden.

Es ist ferner möglich, die Polyurethane mit anderen Filmbildnern bzw. Polymeren im Verhältnis 1:9 bis 9:1 zu kombinieren.

Hierzu können beispielsweise folgende Polymere eingesetzt werden: Polyvinylpyrrolidon, Polyvinylpyrrolidon-Copolymere, wasserlösliche Cellulosederivate, wie Hydroxypropylcellulose, Hydroxypropylmethylcellulose, Methylcellulose, Hydroxyethylcellulose, Acrylat- und Methacrylat-Copolymere, Polyethylenglykole, Polyethylenoxid-Polypropylenoxid-Blockpolymere.

Als Überzugsverfahren lassen sich die gebräuchlichen Verfahren, wie das Coaten in der Wirbelschicht oder im Horizontaltrommelcoater, das Tauchschwertverfahren und das Kesselcoatingverfahren, anwenden. Neben der Anwendung auf Tabletten können die erfindungsgemäßen Polymere auch für das Coating von anderen pharmazeutischen Zubereitungen, wie Granulaten, Pellets, Kristallen oder Kapseln, eingesetzt werden. Die neuen Überzugsmittel werden wie üblich in einer Stärke von 5-200 µm , vorzugsweise 10-100 µm, aufgetragen.

Bei der Verwendung als Bindemittel unterscheidet man je nach Verarbeitungsverfahren zwischen Feucht- und Trockenbindemittel. Letztere werden u.a. bei der Direkttablettierung und bei der Trockengranulation bzw. Kompaktierung verwendet. Hierbei wird das Bindemittel mit dem Wirkstoff und gegebenenfalls weiteren Hilfsstoffen vermischt und anschließend direkttablettiert oder granuliert bzw. kompaktiert.

Im Gegensatz dazu wird bei der Feuchtgranulation die Wirkstoff-Hilfsstoffmischung mit einer Lösung des Bindemittels in Wasser oder einem organischen Lösungsmittel befeuchtet, die feuchte Masse durch ein Sieb gegeben und anschließend getrocknet. Befeuchtung und Trocknung können dabei auch parallel ablaufen, wie z. B. in der Wirbelschichtgranulation.

Für eine optimale Verarbeitung soll das Bindemittel in Lösung niedrigviskos sein, da viskose Lösungen zu inhomogenen Granulaten führen.

Ein Bindemittel soll zu gleichmäßigen, harten, abriebsstabilen Granulaten bzw. Tabletten führen. Insbesondere bei Tabletten kommt der Bruchfestigkeit besondere Bedeutung zu, da sich viele Wirkstoffe schlecht verpressen lassen und somit Tabletten mit unzureichender mechanischer Stabilität ergeben.

Weiterhin soll der Zerfall der Arzneiformen sowie die Freisetzungsgeschwindigkeit der Wirkstoffe durch das Bindemittel nicht nennenswert nachteilig beeinflußt werden.

Die gängigsten Bindemittel sind beispielsweise Polyvinylpyrrolidon, Vinylacetat-Vinylpyrrolidon Copolymere, Gelatine, Stärkekleister, Maltodextrine, hydroxyalkylierte bzw. carboxyalkylierte Cellulosederivate, wie Hydroxypropylmethylcellulose, Methylcellulose, Natriumcarboxymethylcellulose sowie natürliche Gummisorten, wie beispielsweise Gummi Arabicum, Pektin oder Alginat.

Viele dieser Bindemittel weisen in Lösung eine hohe Viskosität auf und sind schlecht verarbeitbar. Durch die hohe Viskosität werden die zu granulierenden Pulverteilchen schlecht und ungleichmäßig benetzt, so daß daraus eine zu geringe Granulatfestigkeit und eine ungünstige Korngrößenverteilung resultieren.

Viele Bindemittel sind zudem hygroskopisch und quellen bei Wasseraufnahme. Dadurch können sich die Granulat- und Tabletteneigenschaften dramatisch verändern.

Überraschenderweise wurde nun gefunden, daß die erfindungsgemäßen Polyurethane über ausgezeichnete Bindemittelwirkungen verfügen und zudem in Konzentrationsbereichen von 0,5 bis 20 Gew.-%, bevorzugt 1 bis 10 Gew.-% der Gesamtmenge der Formulierung den Zerfall nicht nennenswert beeinflussen. Aufgrund des guten Benetzungsverhaltens der Polyurethane kann sich zudem die Freisetzung von schlecht löslichen Wirkstoffen verbessern.

Mit den Polyurethanen als Bindemittel ergeben sich mechanisch außerordentlich stabile und auch über lange Lagerzeiten stabile Granulate bzw. Tabletten.

In den nachfolgenden Beispielen wird die Herstellug und Verwendung der erfindungsgemäßen Polyurethane näher erläutert.

### Beispiel 1

### Herstellung der wäßrigen Polyurethan-Dispersion [Polyurethan D mit folgender Zusammensetzung (Gew.-%): 31,84 % Poly THF (MG 650), 1,95 % Hexandiol 1,6, 2,38 % 1,4-Cyclohexyldimethylol, 41,60 % Isophorondiisocyanat, 22,58 % N-(2-Aminoethyl)-2-aminoethansulfonsäure Na-Salz].

In einem Vierhalskolben, der mit Rührer, Tropftrichter, Thermometer, Rückflußkühler und einer Vorrichtung für das Arbeiten unter Stickstoff ausgestattet war, wurden 1,2 Mol Polytetrahydrofuran (MG 650 g/mol), 0,4 Mol 1,6-Hexandiol und 0,4 Mol 1,4-Cyclohexyldimethylol in Methylethyl-keton (ca. 70 Gew.-%ige Lösung) unter Erwärmen auf eine Temperatur von 50°C unter Rühren gelöst. Anschließend wurden unter Rühren 4,5 Mol Isophorondiisocyanat zugetropft, wobei die Reaktionstemperatur anstieg. Unter Rückfluß wurde das Reaktionsgemisch solang gerührt, bis der NCO-Gehalt des Gemisches praktisch konstant blieb. Die Mischung wurde abgekühlt und mit Aceton bis auf eine etwa 50 Gew-%ige Lösung verdünnt. Danach wurden 2,5 Mol N-(2-Aminoethyl)-2-aminoethansulfonsäure Na-Salz-Lösung (50 Gew.-%ige wäßrige Lösung) bei einer Temperatur < 30°C zugerührt. Die Reaktion wurde noch 30 Minuten bei 50°C weiter gerührt. Anschließend wurde Wasser zugegeben und das organische Lösungsmittel unter vermindertem Druck nach Zugabe von einem Tropfen Silicon-Entschäumer bei ca. 40°C destillativ entfernt. Man erhielt eine stabile Dispersion des Polyurethans. Das Produkt hatte einen K-Wert von 60.

### Beispiel 2

### Herstellung von Propranolol-HCl Filmtabletten (magensaftlöslicher Überzug)

Auf 9 mm gewölbte Tablettenkerne mit 40 mg Propranolol-HCl (Fa. Knoll AG), 195,0 mg Ludipress® (Fa. BASF AG), 12,50 mg Kollidon® VA 64 (Fa. BASF AG) und 2,50 mg Magnesiumstearat wurde in einem Horizontaltrommelcoater (Accela-Cota 24", Fa. Manesty) ein Filmüberzug gemäß folgender Zusammensetzung aufgesprüht:

| | |
|---|---|
| Polyurethan A | 10,0 Gew.-% |
| Lutrol® E 6000 (Fa. BASF AG) | 1,0 Gew.-% |
| Sicovit® rot (Fa. BASF AG) | 1,5 Gew.-% |
| Titandioxid BN 56 (Fa. Kronos) | 3,0 Gew.-% |
| Talkum Pulver (Fa. Riedel de Haen) | 4,5 Gew.-% |
| Wasser | 80,0 Gew.-% |

Zur Herstellung der Sprühdispersion wurde das Polyurethan A und Lutrol® E 6000 in Wasser gelöst, mit Sicovit® rot, Titandioxid und Talkum versetzt und anschließend in einer Korundscheibenmühle homogenisiert. 1260 g (incl. eines Zuschlages von 10 % für Sprühverluste) wurden bei einer Zulufttemperatur von 60°C und einer Sprührate von 30 g/min mit einer 1,0 mm weiten Sprühdüse und einem Sprühdruck von 2,0 bar auf 5 000 g Kerne appliziert. Nach der Sprühung wurde noch 5 min. bei 60°C nachgetrocknet.

Man erhielt glatte, leicht glänzende, rote Filmtabletten mit folgenden Eigenschaften:

| | |
|---|---|
| Aussehen | glatte Oberfläche, schön ausgebildete Gravur |
| Zerfall (künstl. Magensaft) | 6 min. 44 s. |
| Zerfallszeitdifferenz (Filmtablette-Kern) | 1 min. 15 s. |
| Bruchfestigkeit | 115 N |
| Bruchfestigkeitsdifferenz (Filmtablette-Kern) | 46 N |

### Vergleichsbeispiel

Analog Beispiel 2 wurde anstelle von Polyurethan A Pharmacoat® 606 (Hydroxypropylmethylcellulose, Fa. Shin-etsu) eingesetzt.

Folgende Tabletteneigenschaften wurden erzielt:

| | |
|---|---|
| Aussehen | leicht rauhe Oberfläche, zugeschmierte Gravur |
| Zerfall (künstl. Magensaft) | 11 min. 12 s. |
| Zerfallszeitdifferenz (Filmtablette-Kern) | 6 min. 43 s. |
| Bruchfestigkeit | 87 N |
| Bruchfestigkeitsdifferenz (Filmtablette-Kern) | 18 N |

### Beispiel 3

Analog Beispiel 2 wurde Polyurethan E [mit folgender Zusammensetzung (Gew.-%): 28,7 % Poly THF (MG 1000), 6,8 % Hexandiol 1,6, 42,0 % Isophorondiisocyanat, 22,50 % N-(2-Aminoethyl)-2-aminoethansulfonsäure Na-Salz] verarbeitet. Die verwendete Sprühlösung war wie folgt zusammengesetzt:

| | |
|---|---|
| Polyurethan E | 20,0 Gew.-% |
| Lutrol® E 6000 (Fa. BASF AG) | 1,0 Gew.-% |
| Sicovit® rot (Fa. BASF AG) | 1,5 Gew.-% |
| Titandioxid BN 56 (Fa. Kronos) | 3,0 Gew.-% |
| Talkum Pulver (Fa. Riedel de Haen) | 4,5 Gew.-% |
| Wasser | 70,0 Gew.-% |

Es wurden wiederum glatte, leicht glänzende, rote Filmtabletten erhalten.

| | |
|---|---|
| Aussehen: | glatte Oberfläche, schön ausgebildete Gravur |
| Zerfall (künstl. Magensaft): | 6 min. 55 s. |
| Zerfallszeitdifferenz (Filmtablette-Kern): | 1 min. 26 s. |
| Bruchfestigkeit: | 119 N |
| Bruchfestigkeitsdifferenz (Filmtablette-Kern): | 50 N |

### Beispiel 4

### Verwendung als Bindemittel in Glibenclamid-Tabletten

910 g Calciumhydrogenphosphat (Fa. Rhone Poulenc) und 10 g Glibenclamid (Fa. Arzneimittelwerk Dresden) wurden über ein 0,8 mm-Sieb gesiebt und in einem Turbulamischer (Fa. Bachofen) 5 min. gemischt. Diese Pulvermischung wurde in einem Stephanmischer (Fa. Stephan) mit 110 g einer 27 Gew.-%igen wäßrigen Zubereitung des Polyurethans A unter Rühren langsam befeuchtet. Zur vollständigen Durchfeuchtung wurde nach der Zugabe der Bindemittelzubereitung noch 2 min. bei 800 U/min weitergerührt. Die feuchte Masse wurde anschließend durch ein 0,8 mm-Sieb gegeben und auf einer Horde 20 Std. bei 25°C abgetrocknet. Nach der Zugabe von 45 g Kollidon® CL (Fa. BASF) und 5 g Magnesiumstearat (Fa. Bärlocher) erfolgte die Endmischung wiederum im Turbulamischer über 5 min. Diese Tablettiermischung wurde anschließend auf einer Korsch PH 106 Rundläuferpresse (Fa. Korsch) bei 10 kN und 18 kN Preßkraft zu biplanaren, facettierten Tabletten mit 12 mm Durchmesser und 500 mg Gesamtgewicht verpreßt.

| Eigenschaften | 10 kN Preßkraft | 18 kN Preßkraft |
|---|---|---|
| Bruchfestigkeit | 31 N | 66 N |
| Friabilität | 0,19 % | 0,13 % |
| Zerfall | < 15 s. | < 15 s. |

### Vergleichsbeispiel

Die Herstellung erfolgte analog Beispiel 4, jedoch mit Hydroxypropylmethylcellulose (Pharmacoat® 603, Fa. Shin-etsu) als Bindemittel, wobei die Konzentration des Bindemittels in der Lösung aus Viskositätsgründen auf 20 Gew.-% reduziert werden mußte.

| Eigenschaften | 10 kN Preßkraft | 18 kN Preßkraft |
|---|---|---|
| Bruchfestigkeit | 18 N | 45 N |
| Friabilität | 6,3 % | 0,27 % |
| Zerfall | 25 s. | 35 s. |

### Beispiel 5

### Verwendung als Bindemittel in einer Hydrochlorothiazid-Tablette

Eine Mischung aus 8950 g feiner Lactose (Fa. Meggle), 350 g Hydrochlorothiazid (Fa. Chemag) und 350 g Kollidon® CL (Fa. BASF) wird in einem Wirbelschichtgranulationsgerät WSG 15 (Fa. Glatt) mit einer Bindemittelzubereitung bestehend aus 350 g Polyurethan E und 3000 g Wasser besprüht und so in der Wirbelschicht granuliert.

Folgende Prozeßparameter wurden eingestellt:

| | |
|---|---|
| Zulufttemperatur: | 90°C |
| Ablufttemperatur | 37°C |
| Sprührate | 143 g/min |
| Sprühdruck | 4 bar |

Nach der Granulation wurde noch 2,5 min bei 90°C im Gerät nachgetrocknet. Das Granulat wurde über ein 0,8 mm-Sieb gegeben, mit 5 g Magnesiumstearat (Fa. Bärlocher) versetzt und 5 min. in einem Turbulamischer (Fa. Bachofen) gemischt. Die Tablettierung erfolgte auf einer Korsch PH 106 (Fa. Korsch) Rundläuferpresse bei 18 kN Preßkraft zu biplanen, facettierten Tabletten mit 10 mm Durchmesser und 300 mg Gesamtgewicht.

| Granulateigenschaften: | |
|---|---|
| Böschungswinkel | 30° |
| Aussehen | sehr gleichmäßig, ohne nennenswerten Feinanteil |

| Tabletteneigenschaften: | |
|---|---|
| Bruchfestigkeit: | 220 N |
| Friabilität: | < 0,1 % |
| Zerfall: | 4 min. 10 s. |
| Freisetzung im künstl. Magensaft (Ph. Eur.) | 91 % nach 15 min. |

### Vergleichsbeispiel

Die Herstellung erfolgte analog Beispiel 5, jedoch mit Hydroxypropylmethylcellulose (Pharmacoat® 603, Fa. Shin-etsu) als Bindemittel.

| Granulateigenschaften: | |
|---|---|
| Böschungswinkel | 33° |
| Aussehen | etwas ungleichmäßig, teilweise größere Verklumpungen |

| Tabletteneigenschaften: | |
|---|---|
| Bruchfestigkeit: | 175 N |
| Friabilität: | 0,2 % |
| Zerfall: | 5 min. 10 s. |
| Freisetzung im künstl. Magensaft (Ph. Eur.) | 82 % nach 15 min. |

## Patentansprüche

1. Verwendung von wasserlöslichen oder wasserdispergierbaren Polyurethanen aus
a) 0,1 bis 30 Gew.-% mindestens eines Polyols,
b) 20 bis 45 Gew.-% mindestens eines Polyetherpolyols,
c) 10 bis 45 Gew.-% mindestens eines sulfonsäurehaltigen Diamins
d) 30 bis 50 Gew.-% mindestens eines Polyisocyanats sowie gegebenenfalls
e) weiteren Zusatzstoffen
als Überzugsmittel oder Bindemittel für pharmazeutische Darreichungsformen.

2. Verwendung von wasserlöslichen oder wasserdispergierbaren Polyurethanen nach Anspruch 1, die als Komponente a) mindestens ein 2 bis 10 C-Atome enthaltendes Diol enthalten.

3. Verwendung von wasserlöslichen oder wasserdispergierbaren Polyurethanen nach den Ansprüchen 1 und 2, die als Komponente b) mindestens ein Polytetrahydrofuran mit einem Molekulargewicht von 300 bis 6000 g/mol enthalten.

4. Verwendung von wasserlöslichen oder wasserdispergierbaren Polyurethanen nach den Ansprüchen 1 bis 4, die als Komponente d) mindestens ein Diisocyanat der Formel X(NCO)₂ enthalten, wobei X für einen aliphatischen Kohlenwasserstoffrest mit 4 bis 12 Kohlenstoffatomen, einen cycloaliphatischen oder aromatischen Kohlenwasserstoffrest mit 6 bis 15 Kohlenstoffatomen oder einen araliphatischen Kohlenwasserstoffrest mit 7 bis 15 Kohlenstoffatomen steht.

5. Verwendung von wasserlöslichen oder wasserdispergierbaren Polyurethanen nach den Ansprüchen 1 bis 5 mit einem K-Wert von 20 bis 150.

6. Feste pharmazeutische Darreichungsformen mit einem Überzugsmittel aus
a) 0,1 bis 30 Gew.-% mindestens eines Polyols,
b) 20 bis 45 Gew.-% mindestens eines Polyetherpolyols,
c) 10 bis 45 Gew.-% mindestens eines sulfonsäurehaltigen Diamins
d) 30 bis 50 Gew.-% mindestens eines Polyisocyanats sowie gegebenenfalls
e) weiteren Zusatzstoffen.

7. Feste pharmazeutische Darreichungsformen nach Anspruch 6, **dadurch gekennzeichnet, daß** sie das Überzugsmittel neben weiteren üblichen Hilfsstoffen in einer Stärke von 5 bis 200 µm enthalten.

8. Feste pharmazeutische Darreichungsformen nach den Ansprüchen 6 und 7, **dadurch gekennzeichnet, daß** das Überzugsmittel magensaftlöslich oder im Magensaft dispergierbar ist.

9. Feste pharmazeutische Darreichungsformen mit einem Bindemittel aus
a) 0,1 bis 30 Gew.-% mindestens eines Polyols,
b) 20 bis 45 Gew.-% mindestens eines Polyetherpolyols,
c) 10 bis 45 Gew.-% mindestens eines sulfonsäurehaltigen Diamins
d) 30 bis 50 Gew.-% mindestens eines Polyisocyanats sowie gegebenenfalls
e) weiteren Zusatzstoffen.

10. Feste pharmazeutische Darreichungsformen nach Anspruch 9, **dadurch gekennzeichnet, daß** das Bindemittel in einer Konzentration von 0,5 bis 20 Gew.-% enthalten ist.

## Claims

1. The use of water-soluble or water-dispersible polyurethanes consisting of
a) 0.1-30% by weight of at least one polyol,
b) 20-45% by weight of at least one polyetherpolyol,
c) 10-45% by weight of at least one sulfo-containing diamine,
d) 30-50% by weight of at least one polyisocyanate with or without
e) further additives
as coatings or binders for pharmaceutical presentations.

2. The use of water-soluble or water-dispersible polyurethanes as claimed in claim 1, which comprise as component a) at least one diol comprising 2 to 10 carbon atoms.

3. The use of water-soluble or water-dispersible polyurethanes as claimed in claims 1 and 2, which comprise as component b) at least one polytetrahydrofuran with a molecular weight of from 300 to 6000 g/mol.

4. The use of water-soluble or water-dispersible polyurethanes as claimed in any of claims 1 to 4 which comprise as component d) at least one diisocyanate of the formula X(NCO)₂ where X is an aliphatic hydrocarbon radical having 4 to 12 carbon atoms, a cycloaliphatic or aromatic hydrocarbon radical having 6 to 15 carbon atoms or an araliphatic hydrocarbon radical having 7 to 15 carbon atoms.

5. The use of water-soluble or water-dispersible polyurethanes as claimed in any of claims 1 to 5 having a K value of from 20 to 150.

6. A solid pharmaceutical presentation having a coating consisting of
a) 0.1-30% by weight of at least one polyol,
b) 20-45% by weight of at least one polyetherpolyol,
c) 10-45% by weight of at least one sulfo-containing diamine,
d) 30-50% by weight of at least one polyisocyanate with or without
e) further additives.

7. A solid pharmaceutical presentation as claimed in claim 6, which comprises the coating, besides other conventional ancillary substances, in a thickness of from 5 to 200 µm.

8. A solid pharmaceutical presentation as claimed in claims 6 and 7, wherein the coating is soluble in gastric fluid or dispersible in gastric fluid.

9. A solid pharmaceutical presentation having a binder consisting of
a) 0.1-30% by weight of at least one polyol,
b) 20-45% by weight of at least one polyetherpolyol,
c) 10-45% by weight of at least one sulfo-containing diamine,
d) 30-50% by weight of at least one polyisocyanate with or without
e) further additives.

10. A solid pharmaceutical presentation as claimed in claim 9, which comprises the binder in a concentration of from 0.5 to 20% by weight.

## Revendications

1. Utilisation de polyuréthannes hydrosolubles ou dispersables dans l'eau, provenant de
a) 0,1 à 30 % en poids d'au moins un polyol,
b) 20 à 45 % en poids d'au moins un polyétherpolyol,
c) 10 à 45 % en poids d'au moins une diamine contenant de l'acide sulfonique,
d) 30 à 50 % en poids d'au moins un polyisocyanate, ainsi que, éventuellement
e) d'autres additifs,
comme agents de revêtement ou liants pour des formes galéniques pharmaceutiques.

2. Utilisation de polyuréthannes hydrosolubles ou dispersables dans l'eau selon la revendication 1, qui contiennent comme composant a) au moins un diol contenant 2 à 10 atomes de carbone.

3. Utilisation de polyuréthannes hydrosolubles ou dispersables dans l'eau selon les revendications 1 et 2, qui contiennent comme composant b) au moins un polytétrahydrofuranne ayant une masse moléculaire de 300 à 6000 g/mol.

4. Utilisation de polyuréthannes hydrosolubles ou dispersables dans l'eau selon les revendications 1 à 4, qui contiennent comme composant d) au moins un diisocyanate de formule X(NCO)₂, tandis que X désigne un reste hydrocarboné aliphatique ayant 4 à 12 atomes de carbone, un reste hydrocaarboné cycloaliphatique ou aromatique ayant 6 à 15 atomes de carbone ou un reste hydrocarboné araliphatique ayant 7 à 15 atomes de carbone.

5. Utilisation de polyuréthannes hydrosolubles ou dispersables dans l'eau selon les revendications 1 à 5, ayant un indice K de 20 à 150.

6. Formes galéniques pharmaceutiques solides ayant un agent de revêtement provenant de
a) 0,1 à 30 % en poids d'au moins un polyol,
b) 20 à 45 % en poids d'au moins un polyétherpolyol,
c) 10 à 45 % en poids d'au moins une diamine contenant de l'acide sulfonique,
d) 30 à 50 % en poids d'au moins un polyisocyanate, ainsi que, éventuellement
e) d'autres additifs.

7. Formes galéniques pharmaceutiques solides selon la revendication 6, **caractérisées par le fait qu'**elles contiennent l'agent de revêtement, outre d'autres adjuvants classiques, dans une épaisseur de 5 à 200 µm.

8. Formes galéniques pharmaceutiques solides selon les revendications 6 et 7, **caractérisées par le fait que** l'agent de revêtement est soluble dans le suc gastrique ou dispersable dans le suc gastrique.

9. Formes galéniques pharmaceutiques solides ayant un liant provenant de
a) 0,1 à 30 % en poids d'au moins un polyol,
b) 20 à 45 % en poids d'au moins un polyétherpolyol,
c) 10 à 45 % en poids d'au moins une diamine contenant de l'acide sulfonique,
d) 30 à 50 % en poids d'au moins un polyisocyanate, ainsi que, éventuellement
e) d'autres additifs.

10. Formes galéniques pharmaceutiques solides selon la revendication 9, **caractérisées par le fait que** le liant est contenu dans une concentration de 0,5 à 20 % en poids.
